# EUROPÄISCHE PATENTSCHRIFT

(11) **EP 2 536 443 B1**
(45) Veröffentlichungstag und Bekanntmachung des Hinweises auf die Patenterteilung: **11.11.2015**
(21) Anmeldenummer: 11702005.7
(22) Anmeldetag: 27.01.2011
(51) Int. Cl.: A61L 2/26, B65D 25/04, B65D 83/14, A61C 19/00

(54) **System zum Desinfizieren, Sterilisieren und/oder Pflegen von ärztlichen, insbesondere zahnärztlichen Instrumenten**
DEVICE FOR DISINFECTING, STERILIZING AND/OR MAINTAINING MEDICAL, IN PARTICULAR DENTAL INSTRUMENTS
APPAREIL POUR DÉSINFECTER, STÉRILISER ET/OU ENTRETENIR DES INSTRUMENTS MÉDICAUX, NOTAMMENT DENTAIRES

(30) Priorität: 17.02.2010 DE 102010002026
(43) Veröffentlichungstag der Anmeldung: 26.12.2012
(73) Patentinhaber: Kaltenbach & Voigt GmbH, 88400 Biberach (DE)
(72) Erfinder: WIEK, Hans-Dieter, 88454 Hochdorf 2 (DE)
(74) Vertreter: Thun, Clemens
(86) Internationale Anmeldenummer: PCT/EP2011/051124
(87) Internationale Veröffentlichungsnummer: WO 2011/101216

(56) Entgegenhaltungen:
- WO-A1-2009/129902
- DE-U1-202007 000 211
- US-A1- 2001 025 857
- US-A1- 2005 211 724
- US-B1- 6 196 421

## Beschreibung

Die vorliegende Erfindung betrifft ein System umfassend einen Vorratsbehälter zum Bereitstellen von Medien, die zum Desinfizieren, Sterilisieren und/oder Pflegen von ärztlichen Instrumenten vorgesehen ist. Insbesondere sollen mit den Medien zahnärztliche Instrumente in einem Wiederaufbereitungsgerät aufbereitet werden.

Bei ärztlichen oder zahnärztlichen Handstücken handelt es sich um rohrförmige Teile, die der Arzt während der Behandlung als Griffhülse ergreift. Ein üblicherweise in der zahnmedizinischen Praxis verwendetes Handstück ist ein sogenanntes Bohrhandstück, das an seinem vorderen Ende ein Behandlungswerkzeug, insbesondere einen Bohrer trägt und mit seinem hinteren Ende mittels einer Kupplung mit einem Versorgungsschlauch gekoppelt ist. Durch das Handstück erstrecken sich Versorgungsleitungen für Energie zum Antrieb des Behandlungsinstruments sowie Fluidleitungen für Behandlungsmedien, beispielsweise Luft und/oder Wasser. Unterschieden wird oftmals zwischen sogenannten Turbinen-Handstücken, bei denen zur Versorgung einer im vorderen Endbereich angeordneten Turbine Druckluft vorgesehen ist, und sogenannten Motor-Handstücken, welche als Antriebseinheit einen Elektromotor aufweisen.

Zur Aufrechterhaltung der Funktion der Handstücke bedarf es von Zeit zu Zeit einer Pflege, insbesondere der drehbar gelagerten Antriebselemente. Ferner führten die in der zahnärztlichen Praxis immer weiter ansteigenden Hygieneanforderungen dazu, dass eine Aufbereitung von Handstücken in regelmäßigen zeitlichen Abständen zu erfolgen hat. Die erfolgreiche Aufbereitung und Einhaltung der entsprechenden Vorgaben muss hierbei durch den Zahnarzt lückenlos dokumentiert werden, was einen nicht unerheblichen personellen und organisatorischen Aufwand nach sich zieht.

Eine manuelle Wiederaufbereitung zahnärztlicher Handstücke erfolgte bislang dadurch, dass die Instrumente nach Gebrauch am Patienten zunächst sprühdesinfiziert und äußerlich abgewaschen wurden. Eine Innenreinigung der Instrumente wurde hingegen in der Regel nicht durchgeführt. Zwischenzeitlich existieren auf dem Markt allerdings Reinigungs- und Desinfektions-Geräte, in denen die Instrumente aufbereitet werden, bevor sie einer Ölpflege unterzogen werden. Die maschinelle Aufbereitung bringt deutliche Vorteile gegenüber einem manuellen Pflegen der Instrumente mit sich, da nur ein maschinelles Verfahren eine sichere und reproduzierbare Reinigung und Pflege ermöglicht.

Die bislang bekannten Geräte können allerdings in der Regel lediglich für einzelne Aufbereitungsschritte benutzt werden, sodass jeweils separat eine Reinigung, eine Pflege und eine Sterilisation durchgeführt werden muss. Die Gesamtheit der hierfür erforderlichen Geräte nimmt einen relativ großen Platz in Anspruch, wobei für jedes der Geräte jeweils elektrische, pneumatische und fluidische Anschlüsse erforderlich sind. Die Realisierung einer vollständigen maschinellen Aufbereitung zahnärztlicher Instrumente mittels einzelner Geräte ist dementsprechend sehr umständlich und mit einem hohen Kostenaufwand verbunden.

Ein weiterer Nachteil besteht darin, dass die einzelnen Geräte in der Regel nicht untereinander vernetzt sind, weshalb ein Datenaustausch zwischen den Geräten nicht erfolgen kann. Dies führt wiederum zu einem Mehraufwand des Bedienpersonals, da keine durchgängig automatische Dokumentation der Instrumentenaufbereitung erstellbar ist. Ferner müssen in Zwischenschritten die Instrumente von Gerät zu Gerät manuell weiterbefördert werden, was mit einem intensiven Personaleinsatz und einem großen Zeitbedarf verbunden ist.

Aus den zuvor genannten Gründen werden in letzter Zeit vermehrt Geräte eingesetzt, in denen alle Schritte zum Wiederaufbereiten der zahnärztlichen Instrumente durchgeführt werden können. Derartige Geräte erlauben also beispielsweise sowohl ein Desinfizieren bzw. Sterilisieren als auch ein Reinigen und Pflegen der Instrumente. Hierzu sind die Geräte mit verschiedenen Vorratsbehältern verbunden, um die im Laufe der Wiederaufbereitung benutzten Wirkstoffe erhalten zu können. Beispielsweise nutzt das Gerät dann im Rahmen der Wiederaufbereitung ein Reinigungsmittel, ein Desinfektionsmittel sowie ein Pflegeöl. Diese Mittel sind üblicherweise in Form von Spraydosen verfügbar. Der Aufwand und der Platzbedarf zur Anbindung der verschiedenen Spraydosen an das Gerät ist dementsprechend verhältnismäßig groß und aufwendig.

Der Platzbedarf bei Bereitstellung mehrerer Medien kann durch den Einsatz von Behältern reduziert werden, welche in der Lage sind, mehr als ein Medium gleichzeitig aufzunehmen. In diesem Zusammen hang sind Druckbehälter mit zwei Kompartimenten und jeweiligen Auslässen beispielsweise aus der US 6,196,421, der US 2001/025857 A1, der DE 20 2007 000 211 U1 oder der US 2005/211724 A1 bekannt.

Der vorliegenden Erfindung liegt die Aufgabe zugrunde, eine neuartige Anordnung zur Verfügung zu stellen, bei der die Entnahme der Medien aus Behältern weiter optimiert ist.

Die Aufgabe wird durch ein System zum Desinfizieren, Sterilisieren und/oder Pflegen von ärztlichen, insbesondere zahnärztlichen Instrumenten mit den Merkmalen des Anspruchs 1 gelöst. Vorteilhafte Weiterbildungen der Erfindung sind Gegenstand der abhängigen Ansprüche.

Die erfindungsgemäße Lösung beruht auf dem Gedanken, einen Vorratsbehälter zu nutzen, der gleichzeitig mehrere Medien beinhaltet. Dabei ist der Behälter derart ausgeführt, dass die verschiedenen Medien einzeln und wahlweise entnommen werden können und ferner auch die Möglichkeit besteht das Treibmittel erst vor Ort zuführen zu können. Dies wird erfindungsgemäß dadurch ermöglicht, dass Anschlusseinrichtungen des Wiederaufbereitungsgeräts eine Nadel zur Entnahme eines Mediums aus dem Vorratsbehälter sowie eine Nadel zum Zuführen des Treibmittels aufweisen.

Dementsprechend wird erfindungsgemäß ein System zum Desinfizieren, Sterilisieren und/oder Pflegen von ärztlichen, insbesondere zahnärztlichen Instrumenten vorgeschlagen, mit
a) einem Wiederaufbereitungsgerät zum Desinfizieren, Sterilisieren und/oder Pflegen der Instrumente, welches Anschlusseinrichtungen zum Anschließen mindestens eines Vorratsbehälters für von dem Gerät genutzten Medien aufweist, sowie
b) mindestens einem Vorratsbehälter zum Bereitstellen von Medien zum Desinfizieren, Sterilisieren und/oder Pflegen der ärztlichen Instrumenten,
   wobei der Vorratsbehälter als Dose ausgeführt ist, in der ein mit einem ersten Entnahmeelement in Verbindung stehender erster Aufnahmebereich ausgebildet ist, der ein erstes Medium enthält,
   und wobei der erste Aufnahmebereich zumindest teilweise über seinen Umfang hinweg mit einem zweiten Aufnahmebereich in Kontakt ist, der ein zweites Medium enthält und mit einem Anschlusselement zur Entnahme des zweiten Mediums sowie zum Zuführen eines Treibmittels in den zweiten Aufnahmebereich in Verbindung steht,
   wobei die Anschlusseinrichtungen des Wiederaufbereitungsgeräts eine Nadel zur Entnahme des zweiten Mediums aus dem Vorratsbehälter sowie eine Nadel zum Zuführen des Treibmittels aufweisen.

Die Spraydose ist also derart gestaltet, dass zumindest zwei unterschiedliche Medien in unterschiedlichen Aufnahmebereichen angeordnet sind. Diese können jeweils separat der Dose entnommen werden. Der Platzbedarf zur Anbindung der Vorratsbehälter für die Medien an das zuvor beschriebenen System zum Wiederaufbereiten zahnärztlicher Handstücke wird dementsprechend deutlich reduziert.

Vorzugsweise ist das mit dem ersten Aufnahmebereich in Verbindung stehende Entnahmeelement ein Ventil. Der erste Aufnahmebereich kann dann insbesondere durch einen Beutel gebildet sein.

Das zweite Anschlusselement ist vorzugsweise durch einen Gummistopfen gebildet. Das Entnahmeelement für den ersten Aufnahmebereich und das Anschlusselement für den zweiten Aufnahmebereich können dabei an zwei einander gegenüberliegenden Enden der Dose angeordnet sein.

Vorzugsweise sind beide Nadeln konzentrisch zueinander angeordnet. Als Treibmittel kann insbesondere Druckluft verwendet werden, welche durch das System zur Verfügung gestellt wird.

Nachfolgend soll die Erfindung anhand der beiliegenden Zeichnung näher erläutert werden. Es zeigen:
- Figur 1: in Schnittdarstellung eine Prozess- bzw. Spülkammer eines Geräts zum Desinfizieren, Sterilisieren und/oder Pflegen von zahnärztlichen Instrumenten; und
- Figur 2: ein Ausführungsbeispiel eines erfindungsgemäßen Vorratsbehälters.

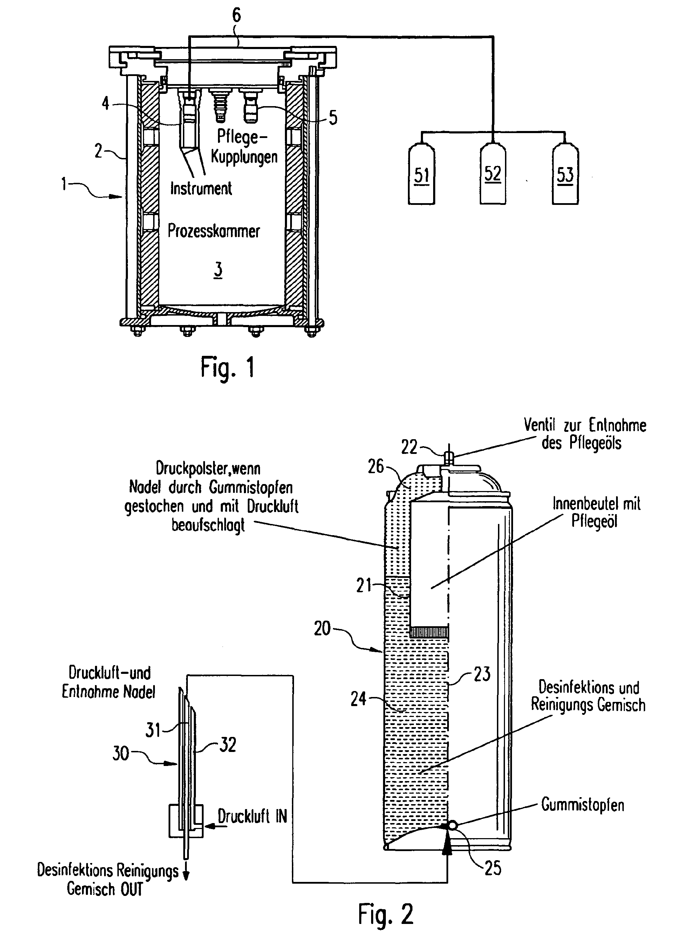

Figur 1 zeigt zunächst schematisch die Ausgestaltung eines Geräts zum Desinfizieren, Sterilisieren und/oder Pflegen von ärztlichen, insbesondere zahnärztlichen Instrumenten, wobei das Gerät nachfolgend allgemein mit dem Bezugszeichen 1 versehen ist. Zentrales Element des erfindungsgemäßen Pflegegeräts 1 ist ein Druckbehälter 2, der eine Prozess- bzw. Spülkammer 3 umschließt. In dieser Spülkammer 3 sind während des Prozessablaufs die zu reinigenden bzw. pflegenden Instrumente 4 angeordnet. Die Anordnung der Instrumente 4 erfolgt hierbei mit Hilfe eines Instrumententrägers, auf dem mehrere Steckplätze bzw. Kupplungen 5 angeordnet sind. Vorzugsweise sind unterschiedliche Kupplungen 5 vorgesehen, sodass Instrumente 4 mit Kupplungssystemen verschiedener Hersteller aufbereitet werden können. Als Instrumententräger dient im vorliegenden Fall der Deckel 6 der Prozesskammer 3. Dieser Deckel 6 stellt die fluidische Ankopplung der zu reinigenden Instrumente 4 an ein Versorgungssystem sicher. Er wird durch eine Verriegelungsvorrichtung auf den Bund des Druckbehälters 2 geklemmt und gegenüber diesem abgedichtet. Über in den Deckel 6 integrierte Verbindungsrohre können dann die einzelnen Instrumente 4 und deren Kanäle einzeln oder gemeinsam mit einem Reinigungs- und/oder Pflegemittel beaufschlagt werden.

Nachfolgend soll zunächst allgemein der Prozessablauf bei der Reinigung und/oder Pflege der Instrumente 4 beschrieben werden. Hierbei wird vor dem Start der Aufbereitung die Druckdichtigkeit der Prozesskammer 3 überprüft. Dabei wird sichergestellt, dass der Deckel 6 richtig eingesetzt und mit dem Druckbehälter 2 verriegelt ist. Auch eine korrekte Verbindung der Fluidleitungen zwischen dem Deckel 6 und in dem Bund des Druckbehälters 2 verlaufenden Leitungen wird überprüft.

Zur Wasserversorgung des Geräts 1 wird Leitungswasser vorzugsweise mittels einer Osmose-Anlage mit oder ohne nachgeschalteten Mischbett-Ionenaustauscher filtriert, wobei die gelösten Salze entfernt werden. Das Wasser bei einer Qualität von <15 µS/cm wird in einen geräteseitigen Vorratsbehälter geleitet, wobei der Füllstand über einen Niveauschalter, der als Schwimmerschalter ausgestaltet ist, und die Güte über einen Leitwertsensor kontrolliert wird. Der Einlass in den Vorratsbehälter ist aus hygienischen Gründen mit einer sogenannten freien Fallstrecke ausgestaltet.

Beim Aufbereiten der Instrumente mit Hilfe des erfindungsgemäßen Geräts werden dann nacheinander die folgenden Schritte ausgeführt:

### a) Reinigen

Zunächst wird Wasser aus dem zuvor beschriebenen Vorratsbehälter in die Prozesskammer 3 geleitet, wobei dies über eine Pumpe oder durch Ansaugen über Vakuum erfolgen kann. In der Prozesskammer 3 wird das Wasser mit Hilfe von Heizelementen auf etwa 45°C aufgeheizt. Dabei wird darauf geachtet, dass die Temperatur nicht oberhalb von 45°C liegt, um ein Koagulieren von Eiweiß zu verhindern. Das Wasser wird ferner mit Hilfe einer Pumpe umgewälzt und über Sprühdüsen, welche an der Mantelfläche des Druckbehälters 2 oder in einem Zentraldom angebracht sind, auf die Außenflächen der Instrumente 4 gerichtet, um diese zu reinigen. Dabei kann das Reinigungswasser durch die Instrumente 4 und/oder die Spraykanäle der Instrumente 4 und/oder zur Außenreinigung durch die Sprühdüsen der Prozesskammer 3 geleitet werden.

Das Aufheizen des Waschmediums kann während der Umwälzung erfolgen, sodass die zu reinigenden Flächen zunächst mit kaltem Waschmedium gereinigt werden. Das Reinigungsmittel kann hierbei in Form von Pulver oder in Tablettenform in die Prozesskammer 3 zugegeben werden oder aus einem entsprechenden Vorratsbehälter zudosiert werden. Das Waschmedium kann dabei aus Tensiden bzw. Phosphaten bestehen und einen ph-Wert oberhalb von 10 aufweisen. Zur Beendigung des Waschvorgangs wird das Wasser aus dem Druckbehälter 2 abgelassen.

### b) Klarspülen - Neutralisation

In einem darauffolgenden Schritt wird dann das Wasser aus dem Vorratsbehälter in die Prozesskammer 3 geleitet und nun auf ca. 45°C bis 60°C aufgeheizt. Während des Umwälzenz des Wassers wird aus einem weiteren Vorratsbehälter Klarspüler bzw. Neutralisator zudosiert. Alternativ kann aufgrund der höheren Temperatur im Vergleich zu dem Schritt a) nunmehr auch eine zweite Komponente einer Reinigungstablette aufgelöst werden. Die Flüssigkeit wird wiederum parallel oder zeitversetzt bzw. im Intervallbetrieb durch die Instrumente 4 und die Spraykanäle geleitet bzw. über die Sprühdüsen auf die Außenflächen der Instrumente 4 gerichtet. Als Klarspüler bzw. Neutralisator kommt insbesondere Phosphorsäureester mit einem ph-Wert von 3 bis 5 zur Anwendung.

Die Flüssigkeit kann wiederum aus dem Druckbehälter in die Kanalisation abgelassen werden oder verbleibt im Behälter, um beim späteren Pflegevorgang aus den Instrumenten 4 austretendes überschüssiges Pflegemittel aufzunehmen bzw. um die ölige Instrumentenaußenfläche mit warmer Flüssigkeit kurz abzuspülen. In diesem Fall wird die Flüssigkeit erst nach dem Pflegevorgang abgelassen, wobei es hilfreich sein kann, die Instrumente 4 mit Druckluft zu beaufschlagen, um ein Eindringen von Sprühwasser in das Innere der Instrumente 4 zu verhindern.

### c) Pflegen

In einem dritten Schritt wird aus einem Pflegemittelvorratsbehälter Pflegemittel in das Instrumenteninnere geleitet, sodass die Getriebe und Lagerstellen geschmiert werden. Das Pflegemittel kann dabei in flüssiger Form als Öl oder aus einer Druckdose in einen Druckluftstrahl injektiert werden. Es ist auch möglich, das Öl über das in der Druckdose enthaltene Treibmittel aufzuschäumen und das Instrumenteninnere mit diesem Öl-Luft-Schaum zu füllen. Die Luftbläschen kollabieren in diesem Fall verhältnismäßig rasch, sodass das Öl im gesamten Instrumenteninneren einen gleichförmigen dünnen Ölfilm bildet. Als Schmierstoffe kommen biologisch abbaubare Fettsäure-Esteröl/Weißöl-Gemische zur Anwendung.

### d) Abspülen

Nachdem zuvor beschriebenen Pflegevorgang können die Instrumente an der Außenfläche mit der noch im Behälter befindlichen Klarspülflüssigkeit abgespült werden. Alternativ hierzu wird über eine Pumpe frisches Wasser aus dem Vorratsbehälter der Prozesskammer 3 zugeführt und über die Sprühdüsen auf die Instrumentenaußenflächen gerichtet.

### e) Sterilisation - Vorvakuum

Zum Sterilisieren der Instrumente wird der Prozesskammer 3 frisches Wasser aus dem Vorratsbehälter zugeführt. In der Prozesskammer 3 ist zur Entlüftung eine Vakuumeinrichtung angeschlossen, wobei der Druck innerhalb der Prozesskammer 3 überwacht bzw. registriert wird.

Mit Hilfe der Vakuumeinrichtung wird die Luft aus der Prozesskammer 3 abgesaugt. Das Vakuum wird durch Aufheizen des Wassers über Heizelemente bis auf Atmosphärendruck abgebaut. Die Prozesskammer 3 wird dann mit Wasserdampf befüllt, wobei sich dieser Vorgang je nach Sterilisationsprogramm mehrmals wiederholen kann.

Das verdampfte Wasservolumen kann bei jedem Vakuumzyklus nachgefüllt werden, wobei alternativ hierzu auch die gesamte für die Dampferzeugung erforderliche Wassermenge gleich zu Beginn des Sterilisationszyklus in die Prozesskammer 3 eingebracht werden kann.

Alternativ zur Dampferzeugung über in der Prozesskammer 3 befindliche Heizelemente kann Wasserdampf zum Druckausgleich bei der Entlüftung bzw. zur Sterilisation auch aus einem außerhalb der Prozesskammer 3 befindlichen Dampfdruckkessel zugeführt werden.

### f) Trocknung und Kühlung

Nach Abschluss der Sterilisation werden die Instrumente 4 getrocknet, in dem der in der Prozesskammer 3 befindliche Wasserdampf zur Kondensation gebracht wird. Dies wird dadurch erzielt, dass die Behälterwand oder in dem Behälter befindliche Elemente gekühlt werden, beispielsweise indem aus dem Vorratshälter entnommenes Wasser durch sie geleitet wird. Dabei kann das Wasser kontinuierlich oder intervallförmig zugeführt werden. Nach Beendigung des Kühlvorgangs wird das Wasser abgeleitet. Da nunmehr innerhalb der Kamer 3 eine Temperatur unterhalb von 50°C vorliegt, kann der Deckel 6 geöffnet werden. Der Aufbereitungszyklus für die Instrumente 4 ist hierdurch abgeschlossen.

Aus der obigen Schilderung ergibt sich, dass mit dem Gerät 1 eine vollautomatische Aufbereitung zahnärztlicher Instrumente ermöglicht wird. Eingriffe durch Bedienpersonal sind nicht erforderlich, sodass ein sehr komfortables System vorliegt. Dabei kann selbstverständlich auch von dem geschilderten Auflauf zum Aufbereiten der Instrumente abgewichen werden.

Dem oben geschilderten Verfahrensablauf ist ferner entnehmbar, dass bei der Aufbereitung der Instrumente verschiedene chemische Substanzen zur Reinigung, Pflege und/oder Desinfektion zum Einsatz kommen. Beispielsweise werden bei der Wiederaufbereitung der Instrumente ein Pflegeöl, ein Desinfektionsmittel sowie ein Reinigungsmittel verwendet, sodass - wie in Figur 1 angedeutet - das Gerät 1 mit zumindest drei Vorratsbehältern 51, 52, 53 für die verschiedenen Medien gekoppelt sein müsste.

Da der Aufwand und Platzbedarf für die Anbindung entsprechender Vorratsbehälter verhältnismäßig groß ist, wird erfindungsgemäß vorgeschlagen, einen neuartigen Vorratsbehälter zu verwenden, der nachfolgend anhand von Figur 2 erläutert werden soll.

Auch bei diesem Vorratsbehälter handelt es sich um eine Spraydose 20, welche nunmehr allerdings derart ausgebildet ist, dass sie mehrere unterschiedliche Medien zur Verfügung stellt. Hierzu ist im Innenraum der Dose 20 zunächst ein Innenbeutel 21 angeordnet, der einen ersten Aufnahmeraum bildet und vorzugsweise mit Pflegeöl gefüllt ist. Dieser Innenbeutel 21 ist mit einem an der Oberseite der Dose 20 befindlichen Ventil 22 zur Entnahme des Pflegeöls verbunden.

In dem Raum zwischen dem Beutel 21 und der Doseninnenwand ist ferner ein zweiter Aufnahmebereich 23 ausgebildet, in dem ein weiteres Medium angeordnet ist. Es handelt sich hierbei beispielsweise um ein Gemisch 24 aus einem Reinigung- und Desinfektionsmittel. Auch die Entnahme dieses Gemisches 24 aus dem zweiten Aufnahmebereich 23 ist ermöglicht, wobei hierfür an der Unterseite der Dose 20 ein Gummistopfen 25 angeordnet ist.

Dieser Gummistopfen 25 wird beim Einsetzten der Dose 20 in das Wiederaufbereitungsgerät 1 durch eine Nadelanordung 30 durchstochen, welche genau genommen aus zwei konzentrisch zueinander angeordneten Nadeln 31 und 32 gebildet ist. Die innere Nadel 31 dient dabei zum Entnehmen des Gemisches 24, während hingegen über die äußere Nadel 32 ein Treibmittel in den Innenraum der Dose 20 eingebracht wird. Als Treibmittel wird insbesondere Druckluft verwendet, was den Vorteil mit sich bringt, dass der Innendruck der Dose 50 auf einem ausgewählten Wert konstant gehalten werden kann, wobei dieser ausgewählte Wert in einem bestimmten Druckbereich variiert werden kann. Ein weiterer Vorteil besteht darin, dass die Dose 50 wegen fehlender entzündlicher Treibgase kein Gefahrengut darstellt, was vor allem beim Transport wesentliche Vorteile mit sich bringt.

Wie bereits erwähnt wird die Druckluft über die Außennadel 32 durch den Gummiverschlussstopfen 25 in das Innere der Dose 20 geleitet. Das sich hierbei ergebende Druckpolster 26 unterstützt dabei sowohl die Entnahme des Pflegeöls aus dem Innenbeutel 21 sowie des Gemisches aus dem zweiten Aufnahmeraum 23. Die Entnahme der verschiedenen Medien sowie die Zuführung der Druckluft sollte selbstverständlich über entsprechende Ventile des Wiederaufbereitungsgeräts 1 gesteuert werden.

Letztendlich wird durch die Erfindung die Möglichkeit geschaffen, mehrere Medien innerhalb eines einzigen Vorratsbehälters anzuordnen, sodass die Handhabung des gesamten Systems erleichtert wird. Durch die Reduzierung der Anzahl der anzuschließenden Vorratsbehälter wird ferner auch eine Verwechslungsgefahr reduziert, was eine weitere Erhöhung der Betriebssicherheit mit sich bringt.

## Patentansprüche

1. System zum Desinfizieren, Sterilisieren und/oder Pflegen von ärztlichen, insbesondere zahnärztlichen Instrumenten (4) mit
a) einem Wiederaufbereitungsgerät (1) zum Desinfizieren, Sterilisieren und/oder Pflegen der Instrumente (4), welches Anschlusseinrichtungen zum Anschließen mindestens eines Vorratsbehälters für von dem Gerät (1) genutzten Medien aufweist, sowie
b) mindestens einem Vorratsbehälter (20) zum Bereitstellen von Medien zum Desinfizieren, Sterilisieren und/oder Pflegen der ärztlichen Instrumenten (4), wobei der Vorratsbehälter (20) als Dose ausgeführt ist, in der ein mit einem ersten Entnahmeelement (22) in Verbindung stehender erste Aufnahmebereich (21) ausgebildet ist, der ein erstes Medium enthält,
**dadurch gekennzeichnet, dass**
der erste Aufnahmebereich (21) zumindest teilweise über seinen Umfang hinweg mit einem zweiten Aufnahmebereich (23) in Kontakt ist, der ein zweites Medium (24) enthält und mit einem Anschlusselement (25) zur Entnahme des zweiten Mediums (24) sowie zum Zuführen eines Treibmittels in den zweiten Aufnahmebereich (23) in Verbindung steht,
wobei die Anschlusseinrichtungen des Wiederaufbereitungsgeräts eine Nadel (31) zur Entnahme des zweiten Mediums (24) aus dem Vorratsbehälter (20) sowie eine Nadel (32) zum Zuführen des Treibmittels aufweisen.

2. System nach Anspruch 1,
**dadurch gekennzeichnet,**
**dass** das mit dem ersten Aufnahmebereich (21) in Verbindung stehende Entnahmeelement (22) ein Ventil ist.

3. System nach Anspruch 1 oder 2,
**dadurch gekennzeichnet,**
**dass** der erste Aufnahmebereich (21) durch einen Beutel gebildet ist.

4. System nach einem der vorherigen Ansprüche,
**dadurch gekennzeichnet,**
**dass** das mit dem zweiten Aufnahmebereich (23) in Verbindung stehende Anschlusselement (25) durch einen Gummistopfen gebildet ist.

5. System nach einem der vorherigen Ansprüche,
**dadurch gekennzeichnet,**
**dass** das Entnahmeelement (22) und das Anschlusselement (25) an zwei einander gegenüberliegenden Enden der Dose angeordnet sind.

6. System nach einem der vorherigen Ansprüche,
**dadurch gekennzeichnet,**
**dass** die beiden Nadeln (31, 32) konzentrisch angeordnet sind.

7. System nach einem der vorherigen Ansprüche,
**dadurch gekennzeichnet,**
**dass** als Treibmittel Druckluft verwendet wird.

## Claims

1. A system for disinfecting, sterilising and/or maintaining medical, in particular dental, instruments (4), having
a) a reconditioning device (1) for disinfecting, sterilising and/or maintaining the instruments (4), which has attachment devices for attaching at least one storage container for media used by the device (1), and also
b) at least one storage container (20) for the provision of media for disinfecting, sterilising and/or maintaining the medical instruments (4), wherein the storage container (20) is in the form of a can in which there is formed a first receiving region (21) which is connected with a first removal element (22) and contains a first medium,
**characterised in that**
the first receiving region (21) is in contact over at least part of its periphery with a second receiving region (23) which contains a second medium (24) and is connected with an attachment element (25) for removal of the second medium (24) and also for delivery of a propellant into the second receiving region (23),
wherein the attachment devices of the reconditioning device have a needle (31) for removal of the second medium (24) from the storage container (20) and also a needle (32) for delivery of the propellant.

2. A system according to claim 1,
**characterised in that**
the removal element (22) connected with the first receiving region (21) is a valve.

3. A system according to claim 1 or 2,
**characterised in that**
the first receiving region (21) is formed by a bag.

4. A system according to one of the previous claims,
**characterised in that**
the attachment element (25) connected with the second receiving region (23) is formed by a rubber stopper.

5. A system according to one of the previous claims,
**characterised in that**
the removal element (22) and the attachment element (25) are arranged at two opposite ends of the can.

6. A system according to one of the previous claims,
**characterised in that**
the two needles (31, 32) are arranged concentrically.

7. A system according to one of the previous claims,
**characterised in that**
compressed air is used as the propellant.

## Revendications

1. Système pour désinfecter, stériliser et/ou entretenir des instruments médicaux, en particulier dentaires (4) avec
a) un appareil de retraitement (1) pour désinfecter, stériliser et/ou entretenir les instruments (4), qui comprend des dispositifs de raccordement pour le raccordement d'au moins un réservoir pour les fluides utilisés par l'appareil (1), ainsi que
b) au moins un réservoir (20) pour fournir des fluides pour désinfecter, stériliser et/ou entretenir les instruments médicaux (4),
le réservoir (20) étant réalisé sous forme de capsule dans laquelle est formée une première zone réceptrice (21) reliée à un premier élément de prélèvement (22) qui contient un premier fluide,
**caractérisé en ce que** la première zone réceptrice (21) est au moins partiellement en contact sur sa périphérie avec une seconde zone réceptrice (23) qui contient un second fluide (24) et qui est reliée à un élément de raccordement (25) pour le prélèvement du second fluide (24) ainsi que pour l'amenée d'un propulseur dans la seconde zone réceptrice (23),
les dispositifs de raccordement de l'appareil de retraitement présentant une aiguille (31) pour le prélèvement du second fluide (24) du réservoir (20) ainsi qu'une aiguille (32) pour l'amenée du propulseur.

2. Système selon la revendication 1,
**caractérisé en ce**
**que** l'élément de prélèvement (22) relié à la première zone réceptrice (21) est une soupape.

3. Système selon la revendication 1 ou 2,
**caractérisé en ce**
**que** la première zone réceptrice (21) est formée par une poche.

4. Système selon l'une des revendications précédentes,
**caractérisé en ce**
**que** l'élément de raccordement (25) relié à la seconde zone réceptrice (23) est formé par un bouchon en caoutchouc.

5. Système selon l'une des revendications précédentes,
**caractérisé en ce**
**que** l'élément de prélèvement (22) et l'élément de raccordement (25) sont agencés à deux extrémités opposées de la capsule.

6. Système selon l'une des revendications précédentes,
**caractérisé en ce**
**que** les deux aiguilles (31, 32) sont agencées de manière concentrique.

7. Système selon l'une des revendications précédentes,
**caractérisé en ce**
**que** de l'air comprimé est utilisé comme propulseur.
